(19) Europäisches Patentamt
European Patent Office
Office européen des brevets

(11) Veröffentlichungsnummer: **0 224 176**
**A1**

# (12) EUROPÄISCHE PATENTANMELDUNG

(21) Anmeldenummer: 86115951.5

(51) Int. Cl.⁴: C07D 295/20

(22) Anmeldetag: 18.11.86

(30) Priorität: 29.11.85 DE 3542230

(43) Veröffentlichungstag der Anmeldung: 03.06.87 Patentblatt 87/23

(84) Benannte Vertragsstaaten: DE FR GB IT

(71) Anmelder: BAYER AG
Konzernverwaltung RP Patentabteilung
D-5090 Leverkusen 1 Bayerwerk(DE)

(72) Erfinder: Heitz, Walter, Prof. Dr.
Am Schmidtborn 5
D-3575 Kirchhain(DE)
Erfinder: Schwalm, Reinhold, Dr.
Am Hüttenwingert 53
D-6706 Wachenheim(DE)

## (54) Verfahren zur Herstellung von Oligomeren und Telechelen mit Carboxy-piperazin-Einheiten und neue Oligomere und Telechele des Poly(carboxypiperazins).

(57) Die Erfindung betrifft ein Verfahren zur Herstellung von Oligomeren und Telechelen des Poly(carboxy-piperazins), sowie neue Oligomere und Telechele des Poly(carboxy-piperazins).

EP 0 224 176 A1

# Verfahren zur Herstellung von Oligomeren und Telechelen mit Carboxy-piperazin-Einheiten und neue Oligomere und Telechele des Poly(carboxypiperazins)

Die Erfindung betrifft ein Verfahren zur Herstellung von Oligomeren und Telechelen des Poly(carboxy-piperazins), sowie neue Oligomere und Telechele des Poly(carboxy-piperazins).

Hochschmelzendes, polymeres Poly(carboxy-piperazin) und seine Herstellung ist z.B. aus US-PS 3 130 179 bekannt. Zum Beispiel aus Aust. J. Chem. 19, 869 (1966) ist bekannt, N,N′-Di(chlorcarbonyl)piperazin mit einer Reihe von Verbindungen umzusetzen, u.a. Phenol, Piperidin, N-Carboxyethylpiperazin. Aus dem Umsetzungsprodukt mit N-Carboxyethylpiperazin entsteht durch Hydrolyse auch das Trimere

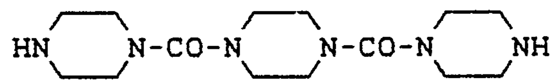 .

Es wurde ein Verfahren zur Herstellung von Oligomeren und Telechelen des Poly(carboxy-piperazins) gefunden, sowie neue Oligomere und Telechele des Poly(carboxypiperazins), die unzersetzt aufgeschmolzen werden können.

Gegenstand der Erfindung ist daher ein Verfahren zur Herstellung von oligomeren und telechelen Poly-(carboxy-piperazinen) der Formel (I)

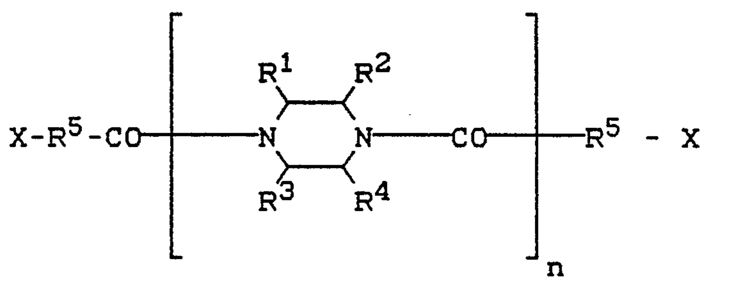 (I),

in welcher

$R^1$, $R^2$, $R^3$ und $R^4$ unabhängig voneinander für Wasserstoff, $C_1$-$C_6$-Alkyl, $C_5$-$C_8$-Cycloalkyl, $C_6$-$C_{10}$-Cycloalkylalkyl, $C_6$-$C_{10}$-Aryl und $C_7$-$C_{14}$-Aralkyl stehen,

$R^5$ für

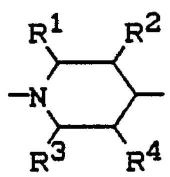 (Piperylen), worin $R^1$-$R^4$ die bei Formel (I) angegebene Bedeutung haben,

und für 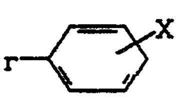, worin

X für Wasserstoff steht und die Bedeutung eines der Reste $R^1$, $R^2$, $R^3$ oder $R^4$ hat, steht und

n für die Zahl 1, 2 oder 3 steht, wobei diejenigen Verbindungen der Formel (I), in der n für die Zahl 2 oder 3 steht, neu sind,

dadurch gekennzeichnet, daß man Piperazin oder Piperazin-N-carbonsäureester der Formel (II)

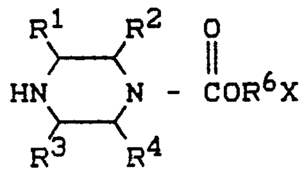

(II),

in welcher
$R^1$ bis $R^4$ die bei Formel (I) angegebene Bedeutung haben,
$R^6$ für $C_1$-$C_{10}$ Alkyl, $C_5$-$C_{10}$ Cycloalkyl, $C_7$-$C_{17}$ Aralkyl, und
X für Wasserstoff steht und die Bedeutung eines der Reste $R^1$, $R^2$, $R^3$ oder $R^4$ hat,
in Gegenwart von tert. Aminen mit Kohlensäureestern der Formel (III)

$R^7-\overset{O}{\overset{\|}{C}}-OR^8-X$ (III),

in welcher
$R^7$ Cl, $-OR^8$-X,
$R^8$ $C_6$-$C_{14}$ Aryl, $C_7$-$C_{20}$ Aralkyl bedeutet und
X für Wasserstoff steht und die Bedeutung eines der Reste $R^1$, $R^2$, $R^3$ oder $R^4$ hat,
in aprotischen Lösungsmitteln bei Temperaturen von 0 bis 200°C, zu N,N'-disubstituierten Piperazinderivaten der Formel (IV)

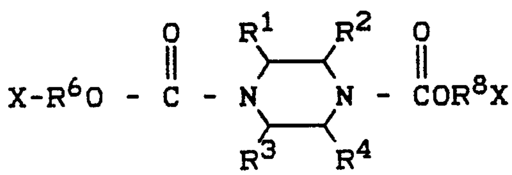

(IV),

in welcher
X für Wasserstoff steht und die Bedeutung eines der Reste $R^1$, $R^2$, $R^3$ oder $R^4$ hat,
$R^6$ die bei Formel (II) oder für $R^8$ angegebene Bedeutung hat,
$R^8$ die bei Formel (III) angegebene Bedeutung hat,
umsetzt und anschließend die aktive Estergruppe $X-R^8-O-\underset{O}{\underset{\|}{C}}-$
mit Piperazin bzw. Piperidinderivaten umsetzt bzw. mit wäßriger (Erd)Alkalilösung bei Temperaturen von 0 bis 100°C die nicht umgesetzte Gruppe

$-\underset{O}{\underset{\|}{C}}-OR^6X$, worin $R^6$ und X die bei Formel (I) und (II) angegebene Bedeutung haben,
abspaltet und das erhaltene Piperazinderivat der Formel (V)

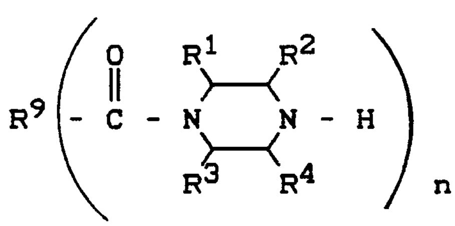

(V),

in welcher
n, $R^1$ bis $R^4$ die bei Formel (I) angegebene Bedeutung haben und
$R^9$ für $R^5$ steht (n = 1), oder für

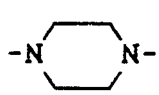

(n = 2), mit Diphenylcarbonat bei Temperaturen von 100 bis 250°C in Gegenwart von tert. Aminen

umsetzt.

Die Verbindungen der Formel V mit

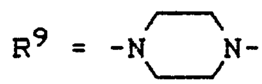

können ebenso mit Säurechloriden

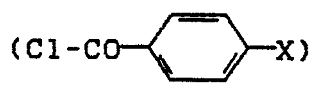

zu Telechelen bzw. die Umsetzungsprodukte mit Diphenylcarbonat ebenfalls weiter mit Piperidinderivaten

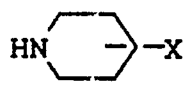

in Gegenwart von tert. Aminen zu Oligomeren (X = H) bzw. Telechelen umgesetzt werden.

Die erfindungsgemäßen Verbindungen sind unzersetzt schmelzbar. Sie zeigen erst oberhalb von 360°C Zersetzung. Aus den Schmelzpunkten der Oligomeren läßt sich der Schmelzpunkt von Poly-(carboxypiperazin) zu ca. 800°C extrapolieren und kann damit über der Zersetzungstemperatur liegen.

Die Oligomeren und Telechelen des Carboxypiperazins haben als Polyharnstoffe von sekundären Diaminen eine außergewöhnliche Hydrolysestabilität und können, da sie hohe Kristallinität aufweisen, bevorzugt als Blöcke in organische Synthesefaser-und Filmmaterialien wie Polyamide und Polyurethane, sowie in Konstruktionsthermoplaste wie aliphatische Polyamide, Polyphenylensulfide usw., eingebaut werden.

Bevorzugte Oligomere und Telechele der Formel (I) sind solche, bei denen $R^1$ bis $R^4$ monovalente $C_1$-$C_6$-Alkylradikale, Wasserstoff, $R^5$-OR-,

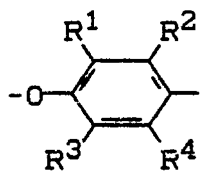

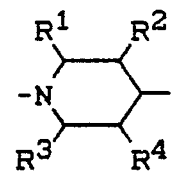

und X = H-, -COOR (R = $C_1$-$C_4$-Alkyl), -COOH, -COCl, -$CH_2OH$, Halogen wie Cl, Br, CN usw. sein kann.

Bevorzugte monovalente Kohlenwasserstoffradikale $R^1$, $R^2$, $R^3$, $R^4$ sind z.B., Alkylradikale wie Methyl-, Ethyl-, n-Propyl-, n-Butyl-, Isobutyl; bevorzugte Cycloalkylradikale sind z.B. Cyclohexyl, Cyclopentyl; bevorzugte Cycloalkylalkylradikale sind z.B. Alkylradikale mit Cycloalkylsubstituenten, z.B. Cyclohexylmethyl-, bevorzugte aromatische Kohlenwasserstoffe ist z.B. Phenyl.

Bevorzugt können Piperazine mit $R^1$, $R^2$, $R^3$, $R^4$, die gleich oder verschieden sein können, wie Wasserstoff oder monovalente Kohlenwasserstoffe mit 1 bis 6 Kohlenstoffatomen, eingesetzt werden.

Beispiele für besonders bevorzugte erfindungsgemäß einzusetzende Piperazine sind 2-Methylpiperazin, 2,5-Dimethylpiperazin, 2-Isobutylpiperazin, 2-Cyclohexylpiperazin, 2-Phenylpiperazin.

Die Reste $R^5$ sind solche wie Alkoxyradikale, die sich von den Bedeutungen der Reste $R^1$, $R^2$, $R^3$, $R^4$ ableiten, z.B. $C_6$-$C_{10}$-Aryloxy wie Phenylen-und Phenylenoxyradikale, Piperidinradikale mit $R_1$ gleich der vorher definierten Bedeutung. Geeignete Gruppen X sind Wasserstoff, -COOR mit R gleich der vorher definierten Bedeutung für $R^1$ bis $R^4$, -COOH, -$R^6$-OH mit $R^6$ = $C_1$-$C_8$-Alkylen, -COCl; Halogen wie Cl, Br, -CN.

Zur Einführung der Reste -$R^5$-X können beispielsweise verwendet werden:

Piperidin, 4-Piperidincarbonsäure, 4-Piperidincarbonsäureethylester, 2-Piperidinmethanol, 2-Piperidinethanol, 3-Piperidinmethanol, p-Chlorphenyl, m-Chlorphenyl, o-Chlorphenyl-, p-Bromphenyl, Phenoxy-, p-Chlorphenoxyradikale.

Die Herstellung der Oligomeren und Telechelen der Formel I mit n = 2 kann mit folgendem Schema wiedergegeben werden:

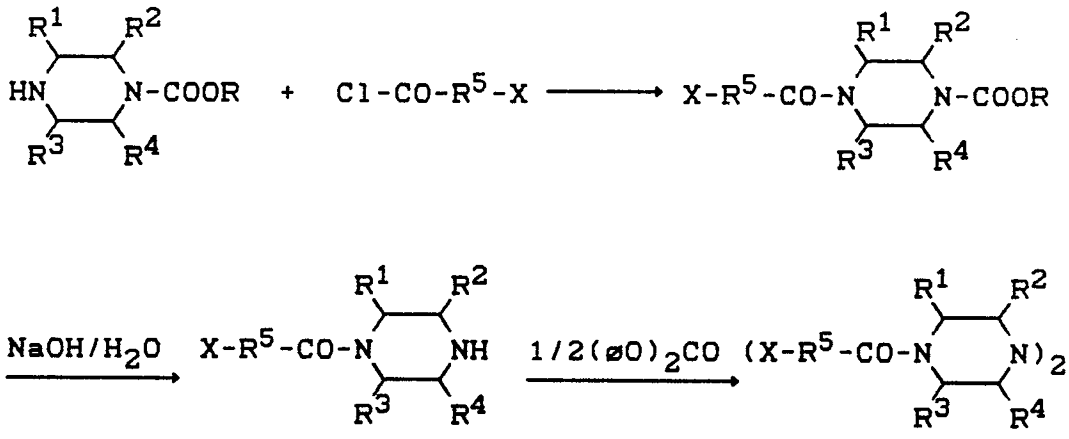


bzw.

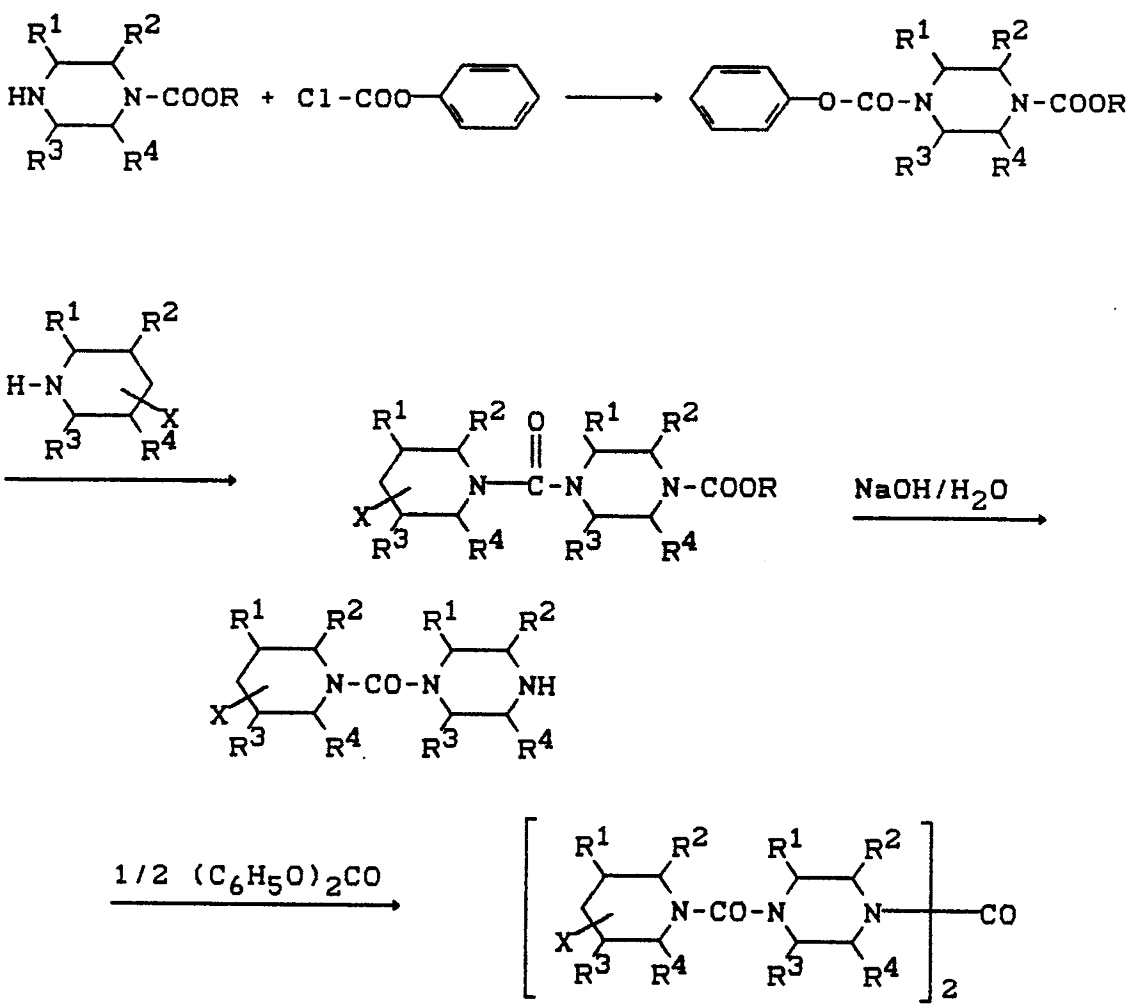


wobei die Reste $R^1$, $R^2$, $R^3$, $R^4$, $R^5$, R, X die bei Formel (I) angegebene Bedeutung haben.

Die Oligomeren und Telechelen der Formel I mit n=3 können nach folgendem Reaktionsschema hergestellt werden:

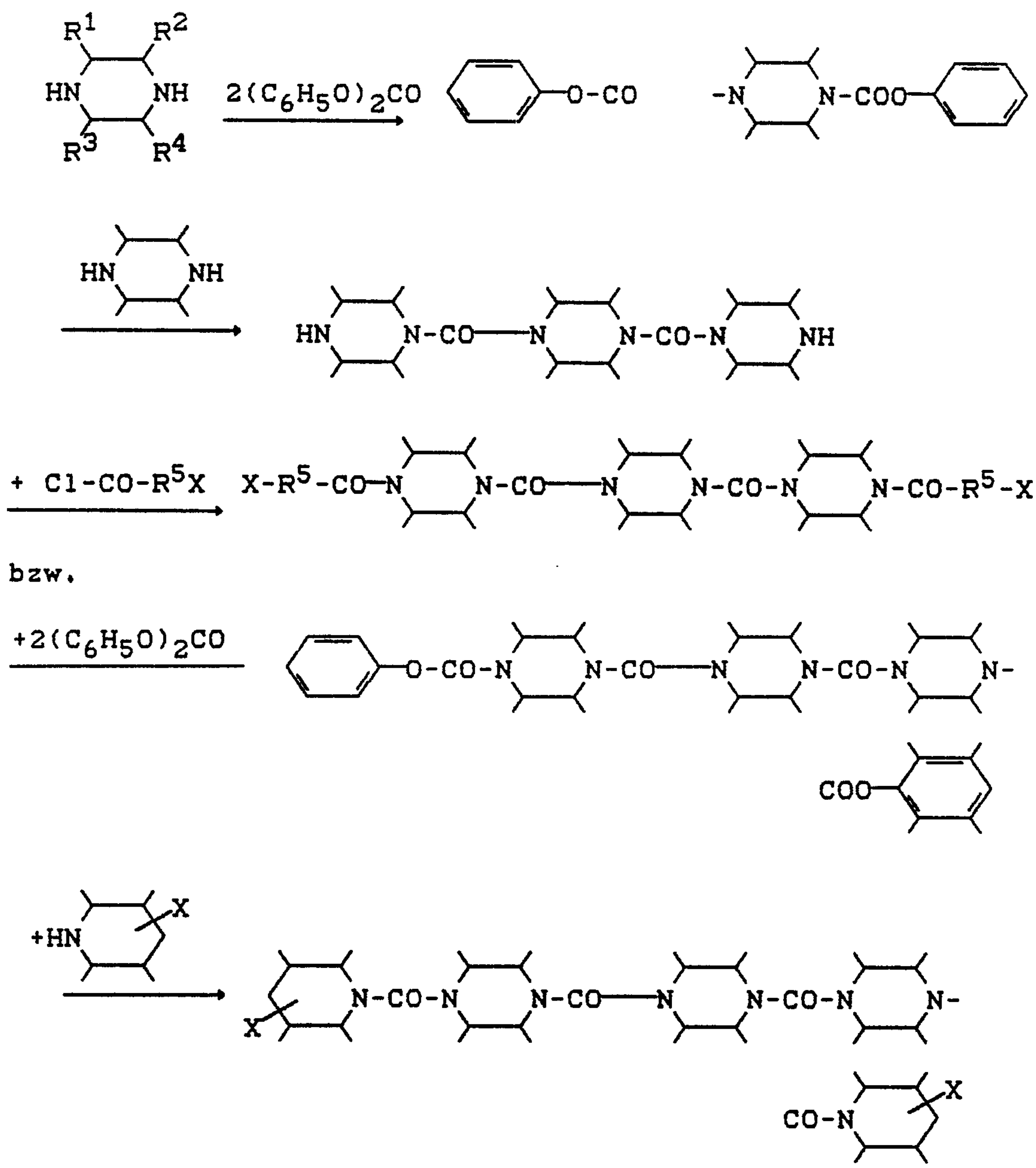


Die erfindungsgemäß hergestellten Telechelen können über ihre reaktiven Endgruppen weiter umgesetzt werden und sie können zum Aufbau von anderen Kunststoffen mitverwendet werden.

Die erfindungsgemäß herstellbaren Oligomeren und Telechelen des Poly(carboxypiperazins) lassen sich im Gegensatz zum Polymeren unzersetzt schmelzen und daher auch zum Aufbau von Polykondensaten verwenden. Sie werden in geeigneter Form z.B. mit Carboxyphenylendgruppen in aliphatische und aromatische Polyester und Polycarbonate, in Polyamide und Polyurethane eingebaut. Sie bilden in diesen Polymeren Hartsegmente, erhöhen die Kristallinität, die Steifigkeit und Härte und tragen bei zur Flammschutzeinstellung dieser Polykondensate.

Zur Herstellung der Oligomeren bzw. Telechelen wurden die Verbindungen der Formel IV in Gegenwart von tert. Aminen mit Piperazin bzw. Piperidinderivaten umgesetzt, so daß alle aktiven aromatischen Estergruppen zu Harnstoffen umgesetzt werden. Ist die Verbindung der Formel IV unsymmetrisch substituiert, wird die verbleibende Alkylestergruppe mit wäßriger Alkalihydroxidlösgung (Konz. 2,1-10 n) bei 0 bis 100°C abgespalten. Die erhaltenen Verbindungen der Formel V werden dann weiter über die sekundäre Amingruppe zu den Oligomeren bzw. Telechelen umgesetzt. Die Umsetzung von gegebenenfalls substituierten Verbindungen der Formel V kann mit Säurechloriden in Gegenwart von tert. Aminen oder mit Diphenylcarbonat im Molverhältnis 1:2 bis 2:1 bei Temperaturen von 0 bis 300°C erfolgen.

Dabei kann in Abwesenheit oder in Anwesenheit von Lösungsmitteln gearbeitet werden. Als Lösungsmittel können beispielsweise verwendet werden; Alkohole, Phenole, Halogenkohlenwaserstoffe, aromatische Kohlenwasserstoffe, Ether, Als tert. Amine können verwendet werden aromatische wie Pyridin, aliphatische wie Triethylamin usw.

Beispiel 1

Herstellung von Poly(carbox-piperazin) mit Piperidin-Endgruppe mit n = 2 (gemäß Formel I)

In einem 250 ml Dreihalskolben mit Rückflußkühler, $N_2$-Einleitungsrohr und Tropftrichter werden 25 g - (150 mmol) Piperazin-N-carbonsäure-ethylester, 30 ml Pyridin und 50 ml trockenes Chloroform vorgelegt und langsam 24,6 g (158 mmol) Chlorameisensäure-phenylester in 50 ml Chloroform bei Raumtemperatur zugetropft, danach wird noch 1 h am Rückfluß erhitzt. Chloroform wird abdestilliert, der Rückstand in Eiswasser gegeben, mit verdünnter Salzsäure angesäuert und das Reaktionsprodukt wird mit Ether extrahiert. Nach Abdestillieren des Ethers bleiben 38,6 g Piperazin-N-carbonsäure-ethyl-N-carbonsäurephenylester zurück (IR: 1723 $cm^{-1}$ >N-CO-O-ø ; 1698 $cm^{-1}$ >N-COOEt).

14,4 g (52 mmol) des Piperazin-N-carbonsäure-ethyl-N'-carbonsäurephenylesters werden mit einem Überschuß Piperidin (10 g = 118 mmol) in einem 100 ml Zweihalskolben mit Rückflußkühler und Innenthermometer zum Sieden erhitzt. Die Innentemperatur steigt im Verlauf von 12 h von 130°C auf 175°C. Die Umsetzung wird mit Hilfe der IR-Spektroskopie verfolgt ( >N-CO-Oø 1723 $cm^{-1}$ verschwindet und eine neue Harnstoffbande N-CO-N bei 1640 $cm^{-1}$ entsteht.

Ausbeute: 9,0 g (64 %).

Das Produkt wurde anschließend verseift. Dazu wurden die erhaltenen 9,0 g des N-Ethoxycarbonylderivats in 250 ml Ethanol mit 200 ml 10 % NaOH fünf Stunden refluxiert.

Ethanol wird abdestilliert, die Lösung mit Kochsalz gesättigt und mit Chloroform extrahiert. Nach dem Trocknen mit $Na_2SO_4$ wird das Chloroform abdestilliert. Es werden 3,1 g (= 48 % Ausbeute) des N-carboxpiperidin-piperazins erhalten.

3,0 g (15 mmol) Piperazin-N-carboxypiperidin werden mit 1,5 g (7 mmol) Diphenylcarbonat und 2,0 g 4-Dimethylaminopyridin als Base in der Schmelze 5 h auf 210°C erhitzt. Nach dem Abkühlen wird die Reaktionsmischung in 10 % NaOH gerührt und der ausgefallene Feststoff abgesaugt. Das IR-Spektrum dieses Feststoffes zeigt neben der Absorptionsbande bei 1640 $cm^{-1}$ noch eine schwache Bande bei 1720 $cm^{-1}$ ( >N-COOø), die vom Monosubstitutionsprodukt mit Diphenylcarbonat stammt. Durch mehrmaliges Extrahieren mit Aceton wird das Nebenprodukt entfernt und das Reaktionsprodukt zweimal aus $CH_2Cl_2$ Hexan umkristallisiert. Es werden 0,96 g (= 33 % der Theorie) des Oligomeren erhalten.

| | | | C | H | N | O |
|---|---|---|---|---|---|---|
| Fp.: | 267° C | | | | | |
| | | ber. | 59,97 | 8,63 | 19,98 | 11,41 |
| | | gef. | 60,12 | 8,35 | 20,00 | 12,03 |

Infrarotabsorptionsbande ($cm^{-1}$)
1639 (C=O Streckschwingung für Harnstoffe)
Massenspektrum: $M^+$ : 420 (45 % bei 28 eV)

Beispiel 2

Herstellung von Poly(carboxy-piperazin) mit Carbonsäurephenylester-Endgruppen (n = 3, gemäß Formel I)

43,0 g (0,5 mmol) Piperazin werden mit 214 g (1 mol) Diphenylcarbonat und 300 ml Ethanol 20 Stunden refluxiert. Nach dem Abkühlen wird abgesaugt und aus Ethanol umkristallisiert. Der entstandene Piperazindiphenylester hat einen Schmelzpunkt von 181 °C.

In einer Apparatur mit 500 ml Dreihalskolben, Rückflußkühler, Thermometer und Feststoffdosiertrichter werden 20 g (61 mmol) des Piperazin-diphenylesters mit Hilfe des Feststoffdosiertrichters innerhalb 8 Stunden zu einem 10-fachen Überschuß (52 g) Piperazin bei 150°C gegeben. Die Reaktionsmischung wird abkühlen lassen und mit verdünnter NaOH über Nacht gerührt. Die Lösung wird mit Kochsalz gesättigt und mit Chloroform extrahiert. Nach dem Trocknen mit $Na_2SO_4$ wird Chloroform abdestilliert und das in geringen Mengen ebenfalls extrahierte Piperazin destillativ entfernt. Das Reaktionsprodukt, 1,4-bis-(piperazinocarbonyl-)piperazin wird aus $CH_2Cl_2$/Hexan umkristallisiert. Fp. 211 °C.

2,0 g (6,5 mmol) 1,4-Bis-(piperazinocarbonyl)-piperazin werden in einem 100 ml Dreihalskolben mit Rückflußkühler und einem Thermometer mit 3,0 g (14 mmol) Diphenylcarbonat in 20 ml Ethanol 20 Stunden refluxiert. Der ausgefallene Feststoff wird aus wäßrigem Aceton umkristallisiert.
Ausbeute: 2,61 g (= 75% der Theorie).

| | | C | H | N | O |
|---|---|---|---|---|---|
| Fp. 248° C | | | | | |
| | ber. | 61,07 | 6,22 | 15,26 | 17,43 |
| | gef. | 611,4 | 6,32 | 15,36 | 17,06 |

Infrarotabsorptionsbanden ($cm^{-1}$)
1639 (C=O Streckschwingung für >N-CO-N< )
1723 (C=O Streckschwingung für >N-CO-O< )

Beispiel 3

Herstellung von Poly(carboxy-piperazin) mit Piperidin-Endgruppen (n = 3, gemäß Formel I)

1,0 g (1,8 mmol) des gemäß Beispiel 2 hergestellten Diphenylethers des 1,4-Bis-(piperazinocarbonyl)-piperazins werden in einem 50 ml Glasautoklaven mit 2,0 g (23,5 mmol) Piperidin 2 Stunden auf 250°C erhitzt. Der Druck steigt dabei bis 6 bar. Das Reaktionsprodukt wird in verdünnter NaOH über Nach gerührt und der ausgefallene Feststoff abgesaugt und zweimal aus $CH_2Cl_2$/Hexan umkristallisiert.

| | | C | H | N | O |
|---|---|---|---|---|---|
| Fp.: 334° C | | | | | |
| | ber. | 58,62 | 8,32 | 21,03 | 12,01 |
| | gef. | 58,97 | 8,34 | 21,05 | 12,09 |

Infrarotabsorptionsbande ($cm^{-1}$)
1639 (C=O Streckschwingung für >N-CO-N< )
Massenspektrum $M^+$ bei m/e 532 (100 % bei 28 eV)

Beispiel 4

Zur Herstellung von Poly(carboxy-piperazin) mit p-Chlorphenyl-Endgruppen werden 4,65 g (15 mmol) des gemäß Beispiel 2 hergestellten 1,4-Bis-(piperazinocarbonyl-)piperazin mit 6,2 g (36 mmol) p-Chlorbenzoylchlorid umgesetzt. Das Säurechlorid wird zusammen mit 3,0 g Pyridin (39 mmol) und 60 ml trockenem $CH_2Cl_2$ vorgelegt und das Amin, gelöst in 40 ml $CH_2Cl_2$, während einer Stunde zugetropft. Es wird eine weitere Stunde refluxiert und über Nach bei Raumtemperatur gerührt. Die Reaktionsmischung wird in Methanol gegeben und das ausgefallene Produkt abgesaugt, mit $NaHCO_3$-Lösung, Wasser und Methanol gewaschen und bei 60°C im Trockenschrank getrocknet. Ausbeute: 6,38 g (= 72,5 %). Anschließend wird aus N-Methylpyrrolidon umkristallisiert.

Fp.: 313° C

| | C | H | N | Cl |
|---|---|---|---|---|
| ber. | 57,23 | 5,49 | 14,3 | 12,06 |
| gef. | 57,22 | 5,57 | 14,28 | 11,98 |

Massenspektrum $M^+$ 586 (87 %)
588 (50 %)
590 (12 %)

## Ansprüche

1. Verfahren zur Herstellung von oligomeren und telechelen Poly(carboxy-piperazinen) der Formel (I)

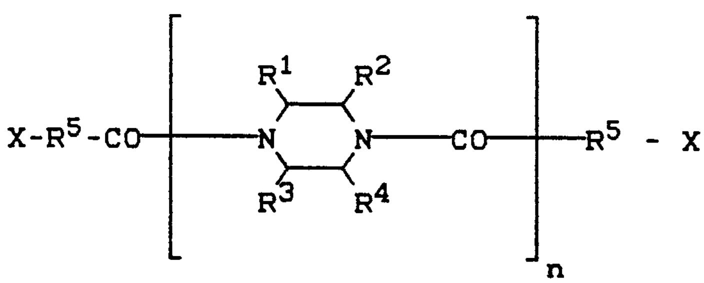
(I),

in welcher
$R^1$, $R^2$, $R^3$ und $R^4$ unabhängig voneinander für Wasserstoff, $C_1$-$C_6$-Alkyl, $C_5$-$C_8$-Cycloalkyl, $C_6$-$C_{10}$-Cycloalkylalkyl, $C_6$-$C_{10}$-Aryl und $C_7$-$C_{14}$-Aralkyl stehen
$R_5$ für

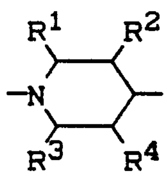
(Piperylen), worin $R^1$-$R^4$ die bei Formel (I) angegebene Bedeutung haben, steht,

und für 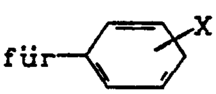 , worin

X für Wasserstoff steht und die Bedeutung eines der Reste $R^1$, $R^2$, $R^3$ oder $R^4$ hat und
n für die Zahl 1, 2 oder 3 steht,

dadurch gekennzeichnet, daß Piperazin oder Piperazin-N-carbonsäureester der Formel (II)

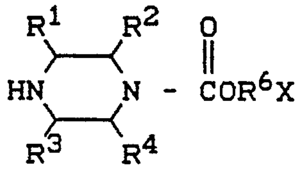

(II),

in welcher
$R^1$ bis $R^4$ die bei Formel (I) angegebene Bedeutung haben,
$R^6$ für $C_1$-$C_{10}$ Alkyl, $C_5$-$C_{10}$ Cycloalkyl, $C_7$-$C_{17}$ Aralkyl, und
X für Wasserstoff steht und die Bedeutung eines der Reste $R^1$, $R^2$, $R^3$ oder $R^4$ hat,
in Gegenwart von tert. Aminen mit Kohlensäureestern der Formel (III)

$R^7 - \overset{\overset{O}{||}}{C} -OR^6 -X$ (III),
in welcher
$R^7$ Cl, $-OR^8$-X,
$R^8$ $C_6$-$C_{14}$ Aryl, $C_7$-$C_{20}$ Aralkyl bedeutet und
X für Wasserstoff steht und die Bedeutung eines der Reste $R^1$, $R^2$, $R^3$ oder $R^4$ hat,
in aprotischen Lösungsmitteln bei Temperaturen von 0 bis 200°C, zu N,N'-disubstituierten Piperazinderivaten der Formel (IV)

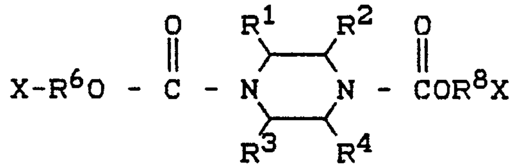

(IV),

in welcher
X für Wasserstoff steht und die Bedeutung eines der Reste $R^1$, $R^2$, $R^3$ oder $R^4$ hat,
$R^6$ die bei Formel (II) oder für $R^8$ angegebene Bedeutung hat,
$R^8$ die bei Formel (III) angegebene Bedeutung hat, umsetzt und anschließend die aktive Estergruppe $X-R^8-O-\underset{\underset{O}{||}}{C}-$
mit Piperazin bzw. Piperidinderivaten umsetzt bzw. mit wäßriger (Erd)Alkalilösung bei Temperaturen von 0 bis 100°C die nicht umgesetzte Gruppe
$-\underset{\underset{O}{||}}{C}-OR^6X$, worin $R^6$ und X die bei Formel (I)
und (II) angegebene Bedeutung haben,
abspaltet und das erhaltene Piperazinderivat der Formel (V)

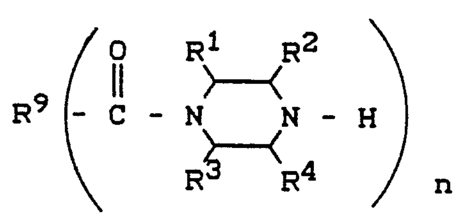

(V),

in welcher
n, $R^1$ bis $R^4$ die bei Formel (I) angegebene Bedeutung haben und
$R^9$ für $R^5$ steht (n = 1), oder für

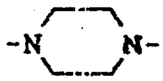

(n = 2),
mit Diphenylcarbonat bei Temperaturen von 100 bis 250°C in Gegenwart von tert. Aminen umsetzt.

2. Gemische von Poly(carboxy-piperazinen) der Formel I mit n = 2 und 3.

3.

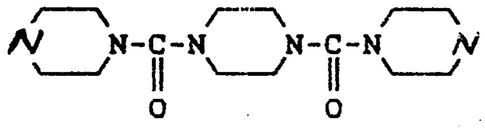

Europäisches Patentamt

# EUROPÄISCHER RECHERCHENBERICHT

Nummer der Anmeldung: EP 86115951.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | AUSTRALIAN JOURNAL OF CHEMISTRY, Band 19, 1966, Melbourne<br>D.E.RIVETT et al. "Some reactions of 1,4-Bischlorocarbonylpiperazine" Seiten 869-875 | | C 07 D 295/20 |
| D,A | * Seite 870, 4. Zeile von unten; Seite 875, Punkt IX * | 1 | |
| D,A | * Seite 873, Zeile 6 *<br>-- | 3 | |
| D,A | US - A - 3 130 179 (R.J.COTTER)<br>* Anspruch 1 *<br>-- | 1 | |
| A | CHEMICAL ABSTRACTS, Band 82, Nr. 17, 28. April 1975, Columbus, Ohio, USA<br>M.I.DOROKLOVA "Synthesis of 1-methyl-piperazine"<br>Seite 511, Zusammenfassung Nr. 112 033n<br>& Khim. Farm. Zh. 1974,8(12),24-7<br>-- | 1 | RECHERCHIERTE SACHGEBIETE (Int. Cl.4)<br>C 07 D 295/00<br>C 07 D 401/00<br>C 07 D 403/00 |
| A | US - A - 3 954 770 (MAYERHOEFER et al.)<br>* Beispiel 1 *<br>-- | 1 | |
| A | GB - A - 772 147 (AMERICAN CYANAMID COMPANY)<br>* Beispiel 1 *<br>-- | 1 | |

Der vorliegende Recherchenbericht wurde für alle Patentansprüche erstellt.

| Recherchenort | Abschlußdatum der Recherche | Prüfer |
|---|---|---|
| WIEN | 25-02-1987 | KÖRBER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

# EUROPÄISCHER RECHERCHENBERICHT

Nummer der Anmeldung

EP 86115951.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US - A - 2 717 896 (GOLDMAN)<br>* Beispiel 1 *<br>-- | 1 | |
| P,X | DIE MAKROMOLEKULARE CHEMIE, Band 187, Nr. 6, Juni 1986<br>R.SCHWALM et al. "Syntheses of telechelic oligo-and poly(carboxy-piperazine)s"<br>Seiten 1415-1422<br>* Seite 1416, Zeile 1 - Seite 1418, Zeile 19 *<br>---- | 1-3 | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 25-02-1987 | KÖRBER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument